# EUROPÄISCHE PATENTSCHRIFT

(11) **EP 1 906 766 B1**
(45) Veröffentlichungstag und Bekanntmachung des Hinweises auf die Patenterteilung: **14.09.2011**
(21) Anmeldenummer: 06776274.0
(22) Anmeldetag: 18.07.2006
(51) Int. Cl.: A23L 1/29, A23L 1/305, A61K 31/205, A23L 1/22, A23L 2/00

(54) **NIEDRIG-GLYKÄMISCHE MISCHUNGEN**
LOW-GLYCEMIC MIXTURES
MELANGES A FAIBLE GLYCEMIE

(30) Priorität: 18.07.2005 DE 102005034043
(43) Veröffentlichungstag der Anmeldung: 09.04.2008
(73) Patentinhaber: Südzucker Aktiengesellschaft Mannheim/Ochsenfurt, 68165 Mannheim (DE)
(72) Erfinder: HAUSMANNS, Stephan, 65185 Wiesbaden (DE); KOWALCZYK, Jörg, 67304 Eisenberg/Steinborn (DE)
(74) Vertreter: Schrell, Andreas
(86) Internationale Anmeldenummer: PCT/EP2006/007041
(87) Internationale Veröffentlichungsnummer: WO 2007/009742

(56) Entgegenhaltungen:
- WO-A1-2004/060081
- WO-A1-2006/094685
- CN-A- 1 555 738
- JP-A- 2004 261 003
- US-A- 5 902 797
- US-A- 5 902 797
- US-A1- 2002 193 342
- DATABASE WPI Week 200661 Derwent Publications Ltd., London, GB; AN 2006-590506 XP002409272 & JP 2006 223224 A (BOURBON KK) 31. August 2006 (2006-08-31)

## Beschreibung

Die vorliegende Erfindung betrifft Gemische, enthaltend L-Carnitin und mindestens ein niedrig-glykämisches Saccharose-Isomer, Produkte enthaltend diese Gemische, Verfahren zu deren Herstellung sowie Verwendungen der Gemische.

L-Carnitin (3-Hydroxy-4(trimethylammonio)-buttersäurebetain) ist eine farblose, in Wasser leicht lösliche Substanz, die vor allem im Fettsäurestoffwechsel als Überträger für Acyl-Gruppen durch die Mitochondrienmembran hindurch dient. Durch die Überführung langkettiger, aktivierter Fettsäuren in die Mitochondrien, wo die Fettsäuren verstoffwechselt werden, kommt L-Carnitin eine bedeutende Rolle bei der Nutzung von Fett zur Energiegewinnung im menschlichen oder tierischen Körper zu. Die beschriebene Rolle von L-Carnitin im Fettstoffwechsel wird in einer Reihe von Nahrungs- und Genussmitteln sowie Getränken genutzt, um Produkte mit gewichtsreduzierender oder zumindest gewichtskonstanthaltender Wirkung bereitzustellen. So beschreibt beispielsweise die WO 97/49304 Sportler- und Energiegetränke, die unter anderem L-Carnitin enthalten und deren Konsum dazu führt, dass energiereiche Substrate wie mittel- und langkettige freie Fettsäuren präferenziell verstoffwechselt werden.

Die beschriebene präferenzielle Nutzung von Fett zur Energiegewinnung im Körper lässt sich jedoch nicht nur durch Nutzung des genannten Effektes von L-Carnitin erreichen, sondern auch auf alternativem Wege. Ein solcher alternativer Weg ist der Einsatz niedrig glykämischer Substanzen, insbesondere niedrig glykämischer Kohlenhydrate. Diese zeichnen sich dadurch aus, dass ihr Konsum zu einem vergleichsweise reduzierten Anstiegs des Insulinspiegels im Körper führt, so dass die Fett- gegenüber der Kohlenhydratoxidation bevorzugt wird. Bekannte niedrig glykämische Zucker sind zum Beispiel Fructose oder Isomaltulose. So sind aus der EP 0 652 012 A oder der WO 97/49304 A bereits Mischungen aus L-Carnitin und Fructose in Sportlergetränken bekannt. CN 15557384 offenbart ein L-Carnitin-haltiges Essiggetränk. Die nach Art. 54 (3) EPÜ nachveröffentlichte WO 2006/094685 A1 offenbart Isomaltulose-haltige und L-Carnitin-haltige Produkte. US 2002/193342 A1 offenbart die Verwendung von Sucralose zur Geschmacksverbesserung von L-Carnitin-haltige Zusammensetzungen.

Die genannten L-Carnitin-haltigen Produkte zeichnen sich allerdings dadurch aus, dass sie vom Konsumenten als geschmacklich beziehungsweise olfaktorisch wenig attraktiv empfunden werden. Ursache dafür ist der eigenartige süßliche Geschmack, verbunden mit einem schwachen Amingeruch von L-Carnitin. L-Carnitin weist bekanntermaßen darüber hinaus eine reduzierte Stabilität auf, wenn es in Mischung mit bestimmten reduzierenden Zuckern, zum Beispiel Fructose, vorliegt. Schließlich zeichnen sich L-Carnitin/Fructose-haltige Getränke durch eine verbesserungsfähige Osmolalität (Isotonie) aus.

Der vorliegenden Erfindung liegt daher das technische Problem zugrunde, Gemische, diese enthaltenen Produkte, Verfahren zu deren Herstellung und deren Verwendungen bereitzustellen, die die vorgenannten Nachteile überwinden, insbesondere L-Carnitin-haltige Gemische bereitzustellen, die bei Verwendung in zum Beispiel Nahrungs-, Genuss-, Arzneimitteln oder Getränken zu einer erhöhten Akzeptanz beim Konsumenten führen, insbesondere geschmacklich und olfaktorisch verbessert und lagerstabiler sind.

Die vorliegende Erfindung löst das ihr zugrundeliegende technische Problem durch die Bereitstellung von Gemischen, enthaltend L-Carnitin und mindestens ein niedrig-glykämisches Saccharoseisomer, wobei das Gemisch 0,05 bis 5 Gewichts-% L-Carnitin und 5 bis 99,9 Gewichts-% des niedrig-glykämischen Saccharoseisomers (jeweils bezogen auf Gesamttrockensubstanz des Gemisches) enthält und wobei das niedrig-glykämische Saccharoseisomer Isomaltulose oder Leukrose ist.

Die vorliegende Erfindung betrifft also Gemische, enthaltend L-Carnitin und mindestens ein niedrig-glykämisches Saccharoseisomer, vorzugsweise bestehend aus L-Carnitin und einem oder zwei niedrig-glykämischen Saccharoseisomeren, das heißt insbesondere bestehend aus L-Carnitin und Leukrose oder bestehend aus L-Carnitin und Isomaltulose oder bestehend aus L-Carnitin und Leukrose und Isomaltulose.

Im Zusammenhang mit der vorliegenden Erfindung wird unter einem niedrig-glykämischen Saccharoseisomer ein Isomer, das heißt ein Strukturisomer von Saccharose verstanden, welches als niedrig-glykämisches Kohlenhydrat im menschlichen Körper wirkt, das heißt nach Konsum in einem vergleichsweise niedrigen respiratorischen Quotienten resultiert. Der respiratorische Quotient spiegelt das Verhältnis von CO₂/O₂ in der Atemluft wieder und ist ein Maß dafür, welche Nährstoffe verbrannt werden. Reine Kohlenhydratverbrennung führt zu einem respiratorischen Quotienten von 1, während reine Fettverbrennung zu einem respriratorischen Quotienten von 0,7 führt. Niedrig-glykämische Kohlenhydrate unterstützen somit passiv die Nutzung von Fett zur Energiegewinnung im Körper, da sie den Insulinspiegel nicht sehr stark ansteigen lassen und damit die Fettoxidation begünstigen.

Im Zusammenhang mit der vorliegenden Erfindung werden unter niedrig-glykämischen Saccharoseisomeren insbesondere Leukrose und Isomaltulose verstanden. Der Begriff "niedrig-glykämisches Saccharoseisomer" im Rahmen der vorliegenden Erfindung steht daher insbesondere dafür, dass a) als niedrig-glykämisches Kohlenhydrat Isomaltulose in dem erfindungsgemäßen L-Carnitin-haltigen Gemisch vorgesehen wird, oder b) Leukrose in dem erfindungsgemäßen L-Carnitin-haltigen Gemisch vorgesehen wird oder c) Leukrose und Isomaltulose, vorzugsweise in einem Verhältnis von 1:99 Gew.-% bis 99:1 Gew.-%, vorzugsweise 30:70 Gew.-% zu 70:30 Gew.-% in dem L-Carnitin-haltigen Gemisch eingesetzt werden (bezogen auf Gesamt-Trockensubstanzgehalt der beiden Isomere).

Die Kombination von L-Carnitin und dem niedrig-glykämischen Saccharoseisomer, insbesondere Isomaltulose und/oder Leukrose, in einem Gemisch führt überraschenderweise dazu, dass der vom Konsumenten als eigentümlich süßlich empfundene Geschmack von L-Carnitin maskiert wird und das erhaltene Gemisch angenehm süß schmeckt, insbesondere auch in damit hergestellten Produkten. Darüber hinaus wird der von vielen Verbrauchern als unangenehm empfundene schwache Amingeruch des L-Carnitins ebenfalls maskiert. Es wurde überraschenderweise gefunden, dass Isomaltulose in wässrigen Lösungen, das heisst in Getränken, erstaunlicherweise die von vielen Konsumenten als nachteilig empfundene Bitterkeit und Adstringenz des L-Carnitins sehr gut maskiert, insbesondere im Vergleich mit Fructose, die, obgleich sie erheblich süßer als Isomaltulose ist, einen erheblich geringeren maskierenden Effekt auf die Adstringenz und die Bitterkeit aufweist. Schließlich konnte überraschenderweise beobachtet werden, dass die Stabilität des L-Carnitins in Anwesenheit von mindestens einem niedrig-glykämischen Saccharoseisomer, insbesondere von Isomaltulose, Leukrose oder einer Kombination von Isomaltulose und Leukrose, insbesondere auch im Vergleich zu L-Carnitin-Fructosemischungen erheblich erhöht wurde. Schließlich zeigt sich eine bessere Osmolarität (Isotonie) von Getränken, die auf der Basis von L-Carnitin- und Leukrose- und/oder Isomaltulose-haltigen Gemischen hergestellt wurden, im Vergleich zu bekannten Monosaccharid-haltigen Getränken, weiche L-Carnitin enthalten.

Im Zusammenhang mit der vorliegenden Erfindung wird unter einem besseren Geschmack verstanden, dass in einem statistisch signifikanten Ausmaß eine Testpersonengruppe den Geschmack eines Isomaltulose- und L-Carnitin-haltigen Produktes als angenehm süß im Unterschied zu dem eigentümlich-süßlichen Geschmack eines L-Carnitin-haltigen Vergleichsprodukts ohne Zusatz von Isomaltulose und/oder Leukrose empfindet.

Im Zusammenhang mit der vorliegenden Erfindung wird unter einem verbesserten Geruch verstanden, dass in einem statistisch signifikanten Ausmaß eine Testpersonengruppe an einem Isomaltulose- und L-Carnitin-haltigen Produkt den schwachen Amingeruch nicht wahrnimmt, der in einem L-Carnitin-haltigen Vergleichsprodukt ohne Zusatz von Isomaltulose und/oder Leukrose wahrgenommen wird.

In bevorzugter Form liegt das efindungsgemäße Gemisch in fester Form, zum Beispiel in körniger, granulärer, agglomerierter, kristalliner (im Falle von insbesondere Isomaltulose), amorpher (im Falle von insbesondere Leukrose), glasartiger oder partikulärer Form vor. Das Gemisch kann auch in flüssiger Form, zum Beispiel gelöst in Wasser vorliegen.

Die vorliegende Erfindung betriff ein vorgenanntes Gemisch, wobei dieses Gemisch 0,05 bis 5 Gew.-% L-Carnitin, vorzugsweise 0,1 bis 5 Gew.-% L-Carnitin, vorzugsweise 0,1 bis 4 Gew.-% L-Carnitin, insbesondere 1 bis 5 Gew.-% L-Carnitin, insbesondere 0,5 bis 3 Gew.-% L-Carnitin enthält (jeweils bezogen auf Gesamttrockensubstanz des Gemisches).

In einer weiteren bevorzugten Ausführungsform betrifft die Erfindung ein vorgenanntes Gemisch, wobei dieses Gemisch 5 Gew.-%, vorzugsweise 10, 20, 30, 40, 50, 60, 70, 80, 90, 95, 96, 97, 98 oder 99 oder 99,5 bis 99,9 Gew.-% niedrig-glykämisches Saccharoseisomer, vorzugsweise 5 bis 80 Gew.-% niedrig-glykämisches Saccharoseisomer, insbesondere 50 bis 75 Gew.-% oder 10 bis 60 Gew.-% niedrig-glykämisches Saccharoseisomer enthält (jeweils bezogen auf Gesamttrockensubstanz des Gemisches).

Die vorliegende Erfindung betrifft insbesondere auch das vorgenannte Gemisch, enthaltend 0,05 bis 5 Gew.-% L-Carnitin und 0,1 Gew.-%, vorzugsweise 10, 20, 30, 40, 50, 60, 70, 80, 90, vorzugsweise 95 Gew.-%, bis 99,95 Gew.-% niedrig-glykämisches Saccharoseisomer, vorzugsweise 0,1 bis 4 Gew.-% L-Carnitin und 0,1 Gew.-%, vorzugsweise 10, 20, 30, 40, 50, 60, 70, 80, 90, vorzugsweise 96 Gew.-%, bis 99,9 Gew.-% niedrig-glykämisches Saccharoseisomer, vorzugsweise 0,1 bis 4 Gew.-% L-Carnitin und 5 Gew.%, vorzugsweise 10, 20, 30, 40, 50, 60, 70, 80, 90, vorzugsweise 96 Gew.-% bis 99,9 Gew.-% niedrig-glykämisches Saccharoseisomer, insbesondere 0,1 bis 4 Gew.-% L-Carnitin und 50 Gew.-%, vorzugsweise 96 Gew.-%, bis 99,9 Gew.-% niedrig-glykämisches Saccharoseisomer oder 0,1 bis 4 Gew.-% L-Carnitin und 10 Gew.-%, vorzugsweise 96 Gew.-%, bis 99,9 Gew.-% niedrig-glykämisches Saccharoseisomer (jeweils bezogen auf Gesamttrockensubstanz des Gemisches).

In einer weiteren bevorzugten Ausführungsform betrifft die Erfindung ein vorgenanntes Gemisch, wobei dieses Gemisch 0,1 bis 5 Gew.-%, vorzugsweise 0,5 bis 4 Gew.-% L-Carnitin zusammen mit 5 Gew.-%,vorzugsweise 10, 20, 30, 40, 50, 60, 70, 80, 90, vorzugsweise 95 Gew.-% bis 99,9 niedrig-glykämisches Saccharoseisomer, bzw. 96 Gew.-% bis 99,5 Gew.-% niedrig-glykämisches Saccharoseisomer, vorzugsweise 0,5 bis 3 Gew.-% L-Carnitin und 97 bis 99,5 Gew.-% niedrig-glykämisches Saccharoseisomer, insbesondere 0,5 bis 4 Gew.-% L-Carnitin und 50 Gew.%, vorzugsweise 96 Gew.-% bis 99,5 Gew.-% oder 0,5 bis 4 Gew.-% L-Carnitin und 10 Gew.-%, vorzugsweise 96 Gew.-% bis 99,5 Gew.-% niedrig-glykämisches Saccharoseisomer (jeweils bezogen auf Gesamttrockensubstanz des Gemisches) enthält.

In einer weiteren bevorzugten Ausführungsform weist das Gemisch der vorliegenden Erfindung neben L-Carnitin und dem mindestens einen niedrig-glykämischen Saccharoseisomer mindestens einen Zusatzstoff auf, insbesondere in einer Menge von 1 bis 99 Gew.-%, vorzugsweise 2 bis 85 Gew.-%, 3 bis 70 Gew.-%, 5 bis 60 Gew.-%, 10 bis 50 Gew.%, 20 bis 40 Gew.-% oder 25 bis 38 Gew.-%, bezogen auf Gesamttrockengewicht des Gemisches.

Unter einem Ergänzungsstoff werden solche Stoffe verstanden, die insbesondere das Aussehen, den Geschmack, die Organoleptik, den Nährwert, ernährungsphysiologische Eigenschaften, die Verarbeitbarkeit, die Lagerfähigkeit oder die Gebrauchsfertigkeit der Mischung beeinflussen.

In einer weiteren bevorzugten Ausführungsform ist vorgesehen, dass der mindestens eine Zusatzstoff ein Präbiotikum ist.

In einer weiteren bevorzugten Ausführungsform ist vorgesehen, dass der mindestens eine Zusatzstoff ein Probiotikum ist.

In einer weiteren bevorzugten Ausführungsform ist vorgesehen, dass der mindestens eine Zusatzstoff ein Ergänzungsstoff ist.

In einer weiteren bevorzugten Ausführungsform ist vorgesehen, dass der mindestens eine Zusatzstoff eine fetthaltige Komponente ist.

In einer weiteren bevorzugten Ausführungsform ist vorgesehen, dass der mindestens eine Zusatzstoff ein Milcherzeugnis ist.

In einer weiteren bevorzugten Ausführungsform ist vorgesehen, dass der mindestens eine Zusatzstoff ein süßendes Agens ist.

Im Zusammenhang mit der vorliegenden Erfindung werden unter dem Begriff "süßendes Agens" Substanzen verstanden, die Süßkraft aufweisen und zum Beispiel Lebensmitteln oder Getränken zugesetzt werden, um einen Süßegeschmack hervorzurufen. Im Zusammenhang mit der vorliegenden Erfindung werden die "süßenden Agenzien" unterteilt in "Zucker" wie Isomaltulose, Saccharose, Glucose oder Fructose, die Körper und Süßkraft geben sowie "Süßungsmittel", also Stoffe, die keine Zucker sind aber trotzdem Süßkraft aufweisen, welche wiederum untergliedert werden in "Zuckeraustauschstoffe", also süßende Agenzien, die einen Körper und einen physiologischen Brennwert zusätzlich zu einer Süßkraft aufweisen (körpergebende Süßungsmittel), und "Intensivsüßstoffe", also Stoffe, die in der Regel eine sehr hohe Süßkraft, aber keinen Körper und in der Regel keinen oder nur einen geringfügigen physiologischen Brennwert aufweisen. Ein Intensivsüßstoff ist beispielsweise Sucralose.

In einer besonders bevorzugten Ausführungsform ist daher das süßende Agens ein Zucker, ein Intensivsüßstoff oder ein Zuckeraustauschstoff.

In einer weiteren bevorzugten Ausführungsform ist der Intensivsüßstoff ausgewählt aus der Gruppe bestehend aus Sucralose, Natriumcyclamat, Acesulfam K, Neohesperidin-Dihydrochalkon, Glycyrrhizin, Steveosid, Monellin, Thaumatin, Aspartam, Dulcin, Saccharin, Naringin-Dihydrochalkon, Neotame und einer Mischung zweier oder mehrerer davon. In einer weiteren bevorzugten Ausführungsform ist der Zuckeraustauschstoff ausgewählt aus der Gruppe bestehend aus Isomalt, 1,1-GPM (1-O-α-D-Glucopyranosyl-D-mannit), 1,6-GPS (6-O-α-D-Glucopyranosyl-D-sorbit), 1,1-GPS (1-O-α-D-Glucopyranosyl-D-sorbit), Maltodextrine, Lactit, Maltit, Erythrit, Xylit, Mannit, Sorbit, Maltitsirup, hydrierte und nichthydrierte Stärkehydrolysate und einer Mischung zweier oder mehrerer davon.

In einer weiteren bevorzugten Ausführungsform ist der mindestens eine Zusatzstoff ein Zucker, insbesondere Saccharose, Glukose, Fructose, Lactose, Maltose oder ein Gemisch zweier oder mehrere davon.

Selbstverständlich betrifft die Erfindung auch die Gemische enthaltend L-Carnitin und das mindestens eine niedrig-glykämische Saccharoseisomer, wobei das mindestens eine niedrig-glykämische Saccharoseisomer das einzig und alleinig in dem Gemisch vorkommende körpergebende süßende Agens ist. In einer weiteren bevorzugten Ausführungsform betrifft die Erfindung ein vorgenanntes Gemisch enthaltend L-Carnitin und das mindestens eine niedrig-glykämische Saccharoseisomer, wobei das mindestens eine niedrig-glykämische Saccharoseisomer der einzig und alleinige in dem Gemisch vorkommende Zucker ist. In einer weiteren bevorzugten Ausführungsform betrifft die vorliegende Erfindung ein Gemisch enthaltend L-Carnitin und das mindestens eine niedrig-glykämische Saccharoseisomer, wobei das mindestens eine niedrig-glykämische Saccharoseisomer das einzig und alleinig in dem Gemisch vorkommende sü-βende Agens ist.

Insbesondere betrifft die Erfindung ein Gemisch enthaltend oder bestehend aus L-Carnitin und das beziehungsweise dem mindestens eine beziehungsweise einem niedrig-glykämische/en Saccharoseisomer und ein Produkt enthaltend dieses Gemisch, wobei das Gemisch und/oder das Produkt diabetikergeeignet ist.

In einer weiteren bevorzugten Ausführungsform betrifft die vorliegende Erfindung ein vorliegendes Gemisch enthaltend L-Carnitin und das mindestens eine niedrig-glykämische Saccharoseisomer, wobei dieses Gemisch frei ist von Saccharose, frei ist von Glucose, frei ist von Lactose, frei ist von Fructose, frei ist von Sorbit, frei ist von Xylit, frei ist von Mannit oder frei ist von einem oder mehreren oder allen der genannten Zucker oder Zuckeralkohole.

In einer weiteren bevorzugten Ausführungsform betrifft die vorliegende Erfindung ein Gemisch enthaltend L-Carnitin und das mindestens eine niedrig-glykämische Saccharoseisomer, wobei dieses als Zusatzstoff ein Präbiotikum, insbesondere Inulin, Oligofructose oder Galactooligosaccharide, enthält.

In einer weiteren bevorzugten Ausführungsform betrifft die vorliegende Erfindung ein Gemisch enthaltend L-Carnitin und das mindestens eine niedrig-glykämische Saccharoseisomer, wobei der mindestens eine Zusatzstoff ein Milcherzeugnis, insbesondere ein lactosefreies Milcherzeugnis ist, zum Beispiel Magermilchpulver, Vollmilchpulver, lactosefreies Magermilchpulver, lactosefreies Vollmilchpulver, ein Molkeerzeugnis oder eine Mischung zweier oder mehrerer davon.

In einer weiteren bevorzugten Ausführungsform betrifft die vorliegende Erfindung ein Gemisch enthaltend L-Carnitin und das mindestens eine niedrig-glykämische Saccharoseisomer, wobei der mindestens eine Zusatzstoff ein Ergänzungsstoff ist, insbesondere ausgewählt aus der Gruppe bestehend aus Malzextrakt, Aromastoffen, Farbstoffen, Geschmacksstoffen, Fließmittel, Mineralstoffen wie Natrium und Kalzium, insbesondere Salzen wie Natriumchlorid, Vitaminen, Folsäure, Emulgatoren, Ballaststoffen, Lecithin, Omega-3-Fettsäuren, Triglyzeride mittlerer Kettenlänge, Phythoöstrogene und Ascorbinsäuresalze.

In einer weiteren bevorzugten Ausführungsform betrifft die Erfindung ein Gemisch enthaltend L-Carnitin und das mindestens eine niedrig-glykämische Saccharoseisomer, wobei als Zusatzstoff ein Probiotikum, zum Beispiel Lactobakterien oder Bifidobakterien, vorhanden ist.

Die Erfindung betrifft vorzugsweise in einer besonderen Ausführungsform eines der vorliegenden Gemische, wobei dieses frei ist von Garcinia-Extrakt, das heisst insbesondere frei von einem Extrakt aus der Rinde von Garcinia, zum Beispiel Garcinia cambogia, Garcinia indica, Garcinia atrovirides oder ähnlichem.

In einer weiteren bevorzugten Ausführungsform ist vorgesehen, dass das erfindungsgemäße Gemisch frei ist von Koffein.

In einer besonders bevorzugten Ausführungsform der vorliegenden Erfindung ist vorgesehen, dass das erfindungsgemäße Gemisch frei ist von Koffein und Garcinia-Extrakt.

Die Erfindung betrifft auch Produkte, enthaltend mindestens eines der vorgenannten Gemische und vorzugsweise mindestens einen der vorstehend ausgeführten Zusatzstoffe, das heißt zum Beispiel ein Präbiotikum, ein Probiotikum, einen Ergänzungsstoff, eine fetthaltige Komponente, ein Milcherzeugnis oder ein süßendes Agens.

In besonders bevorzugter Ausführungsform sind die Produkte feste oder flüssige Produkte.

In einer besonders bevorzugten Ausführungsform der vorliegenden Erfindung ist das Produkt ein flüssiges Produkt, insbesondere ein Produkt enthaltend mindestens eines der vorgenannten Gemische und ein Lösemittel, zum Beispiel Wasser oder Milch. Die Getränke können alkoholische oder nicht-alkoholische Getränke sein. Die Getränke können in einer bevorzugten Ausführungsform diabetikergeeignet sein. In besonders bevorzugter Ausführungsform ist das Produkt ein Getränk, zum Beispiel ein Sportlergetränk, ein Energiegetränk, eine enterale Formulierung, ein Erfrischungsgetränk, ein Cola-haltiges Getränk oder Ähnliches.

In einer besonders bevorzugten Ausführungsform ist vorgesehen, dass das Getränk 2 bis 40 Gew.-%, insbesondere 3 bis 35 Gew.-%, insbesondere 4 bis 30 Gew.-%, insbesondere 10 bis 30 Gew.-% des Gemisches (jeweils bezogen auf das Gesamtgewicht des Getränkes) enthält.

In einer weiteren bevorzugten Ausführungsform ist das Gemisch der vorliegenden Erfindung in einem Produkt enthalten, welches als Nahrungs-, Genuss- oder Arzneimittel ausgeführt ist.

Erfindungsgemäß bevorzugt ist die Verwendung des erfindungsgemäßen Gemisches in einem Nahrungs-, Genuss- oder Arzneimittel, wobei das Nahrungs-, Genuss- oder Arzneimittel 20 bis 99 Gew.-%, insbesondere 20 bis 70 Gew.-%, bevorzugt 30 bis 60 Gew.%, mehr bevorzugt 40 bis 55 Gew.-%, (bezogen auf die GesamtTrockensubstanz des Nahrungs-, Genuss- oder Arzneimittels) des Gemisches enthält. In besonderer Ausführungsform ist das Nahrungs-, Genuss- oder Arzneimittel frei von Glucose, Fructose, Lactose, Saccharose, Sorbit, Xylit und/oder Mannit. Erfindungsgemäß kann es jedoch auch Glucose, Fructose, Saccharose und/oder andere süßende Agenzien enthalten.

Im Zusammenhang mit der vorliegenden Erfindung werden unter dem Begriff "Nahrungsmittel" vorwiegend der menschlichen Ernährung dienende Erzeugnisse oder Stoffgemische in fester, flüssiger, gelöster oder suspendierter Form verstanden, die dazu vorwiegend bestimmt sind, in unverändertem, zubereitetem oder verarbeitetem Zustand vom Menschen verzehrt zu werden. Nahrungsmittel können neben ihren natürlichen Bestandteilen weitere Komponenten enthalten, die natürlicher oder synthetischer Herkunft sein können. Nahrungsmittel können sowohl in fester Form als auch in flüssiger Form vorliegen. Unter einem "Genussmittel" werden vorwiegend dem beim Verzehr auftretenden Genuss des menschlichen oder tierischen Körpers dienende Stoffe oder Stoffgemische in fester, flüssiger, gelöster oder suspendierter Form verstanden.

In bevorzugter Ausführungsform der Erfindung handelt es sich bei den erfindungsgemäßen Nahrungsmitteln um Milcherzeugnisse oder Milchprodukte, beispielsweise Käse-, Butter-, Joghurt-, Kefir-, Quark, Sauermilch-, Buttermilch-, Sahne-, Kondensmilch-, Trockenmilch-, Molken-, Milchmisch-, Milchhalbfett-, Molkenmisch- Milchzucker-, Milcheiweiß- und Milchfett-Produkte. In einer weiteren bevorzugten Ausführungsform der Erfindung handelt es sich bei den erfindungsgemäßen Nahrungsmitteln um Backwaren, insbesondere Brot einschließlich Kleingebäck und feine Backwaren einschließlich Dauer-Backwaren. In weiteren Ausführungsformen der Erfindung handelt es sich bei den erfindungsgemäßen Nahrungsmitteln um Brotaufstriche, Margarine-Erzeugnisse und Backfette sowie Instantprodukte und Brüherzeugnisse. In weiteren bevorzugten Ausführungsformen der Erfindung handelt es sich bei den erfindungsgemäßen Nahrungsmitteln um Obstprodukte, insbesondere Konfitüren, Marmeladen, Gelees, Obstkonserven, Fruchtpulpe, Fruchtmark, Fruchtsäfte, Fruchtsaft-Konzentrate, Fruchtnektar und Fruchtpulver. Die die erfindungsgemäßen Produkte enthaltenen Nahrungsmittel können erfindungsgemäß auch Gemüseerzeugnisse, insbesondere Gemüsekonserven, Gemüsesäfte und Gemüsemark sein. Ein Nahrungsmittel im Sinne der vorliegenden Erfindung kann auch ein Getränkepulver, zum Beispiel ein Instantgetränkepulver sein, zum Beispiel eine Kakao-, Tee- oder Kaffeprodukt-Getränkepulver.

Unter dem Begriff Genussmittel werden beispielsweise Süßwaren, insbesondere Schokoladen-Erzeugnisse, Hartkaramellen, Weichkaramellen, Fondant-Erzeugnisse, Gelee-Erzeugnisse, Lakritzen, Schaumzuckerwaren, Kokosflocken, Dragees, Komprimate, kandierte Früchte, Krokant, Nougaterzeugnisse, Eiskonfekt, Marzipan, Kaugummi, Müsliriegel, sowie Speiseeis verstanden.

Im Zusammenhang mit der vorliegenden Erfindung werden unter "diätetischen Nahrungsmitteln beziehungsweise Getränken" Nahrungsmittel beziehungsweise Getränke verstanden, die bestimmt sind, einem bestimmten Ernährungszweck dazu zu dienen, dass sie die Zufuhr bestimmter Nährstoffe oder anderer ernährungsphysiologisch wirkender Stoffe in einem bestimmten Mengenverhältnis oder in bestimmter Beschaffenheit bewirken. Diätetische Nahrungsmittel oder Getränke unterscheiden sich maßgeblich von Nahrungsmitteln oder Getränken vergleichbarer Art durch ihre Zusammensetzung oder durch ihre Eigenschaften. Diätetische Nahrungsmittel können in Fällen eingesetzt werden, wo bestimmte Ernährungsanforderungen aufgrund von Krankheiten, Funktionsstörungen oder allergischen Reaktionen gegen einzelne Nahrungsmittel beziehungsweise deren Inhaltsstoffe erfüllt werden müssen. Diätetische Nahrungsmittel können sowohl in fester Form als auch in flüssiger Form (Getränk) vorliegen.

Die Erfindung betrifft auch Verfahren zur Herstellung der vorgenannten Gemische und Produkte. Erfindungsgemäß ist daher vorgesehen, ein Verfahren zur Herstellung der vorgenannten Gemische bereitzustellen, in dem L-Carnitin und das mindestens eine niedrig-glykämische Saccharoseisomer bereitgestellt und miteinander im gewünschten Verhältnis vermischt werden.

In einer weiteren bevorzugten Ausführungsform ist vorgesehen, ein dieses Gemisch enthaltendes Produkt herzustellen, in dem die gewünschten Bestandteile dieses Produktes zusammen mit L-Carnitin und dem mindestens einen niedrig-glykämischen Saccharoseisomer bereitgestellt, miteinander in Kontakt gebracht werden und unter den für den Fachmann an sich bekannten Herstellbedingungen das gewünschte Produkts erhalten wird.

In einer weiteren bevorzugten Ausführungsform betrifft die vorliegende Erfindung die Verwendung von dem mindestens einem niedrig-glykämischen Saccharoseisomer, insbesondere Isomaltulose und/oder Leukrose, in einem L-Carnitin-haltigen Produkt zur Geschmacksverbesserung oder Geschmacksmaskierung des in dem Produkt vorhandenen L-Carnitins.

Die Erfindung betrifft auch die Verwendung von mindestens einem niedrig-glykämischen Saccharoseisomer, insbesondere Isomaltulose und/oder Leukrose, in einem L-Carnitin-haltigen Produkt zur Erhöhung der Stabilität von Carnitin in dem Produkt, insbesondere in einem Getränk.

Weitere vorteilhafte Ausgestaltungen der Erfindung ergeben sich aus den Unteransprüchen.

Die Erfindung wird anhand der folgenden Beispiele und der dazugehörigen Figur näher erläutert.

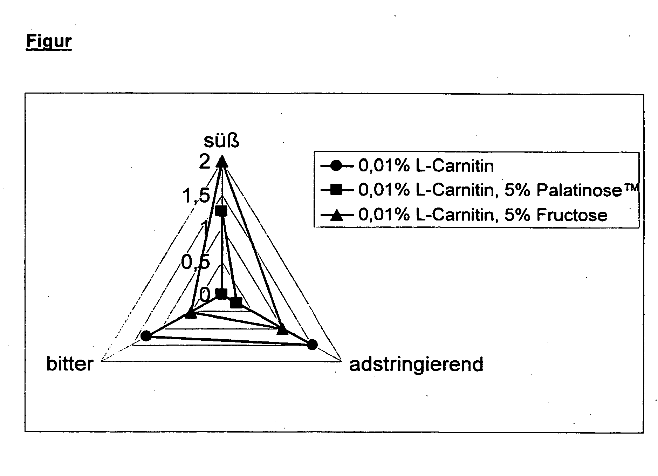

Die Figur zeigt das Geschmacksprofil wässriger Lösungen mit Isomaltulose (Palatinose™) und L-Carnitin sowie von Vergleichslösungen.

### Beispiel 1

Geschmacksprofil wässriger Lösungen mit L-Carnitin

### Proben:

| | |
|---|---|
| Probe 1: | 0,01% L-Carnitin |
| Probe 2: | 0,01% L-Carnitin, 5% Palatinose™ |
| Probe 3: | 0,01% L-Carnitin; 5% Fructose |

### Ergebnis:

Die Proben wurden von erfahrenen Probanden verkostet. Es wurden dabei folgende statistisch abgesicherte in der Figur dargestellte Aussagen erhalten. Mit signifikanter Auffälligkeit wurde festgestellt, dass die wässrige Lösung mit L-Carnitin als einzigem geschmacklich wirkenden Bestandteil deutlich bitterer und adstringierender als die beiden anderen mit Zuckern versehenen Lösungen ist. Erstaunlicherweise stellt sich die Isomaltulose (Palatinose™)-haltige L-Carnitin Lösung als eine Lösung dar, die sowohl die Bitterkeit als auch die adstringierende Komponente des L-Carnitins deutlich besser maskiert als Fructose, obwohl die Lösung, die Fructose enthält, deutlich süßer ist, als die Lösung, die Isomaltulose enthält. Der maskierende Effekt der Isomaltulose kann daher nicht, beziehungsweise nicht allein, auf der erhöhten Süßkraft der Fructose beruhen.

### Beispiel 2

### Stabilität von L-Carnitin-haltigen Isomaltuloselösungen

Hinsichtlich chemischer Stabilität von Isomaltuloselösungen mit Carnitin wurden Erhitzungstests durchgeführt. Als Vergleich wurde dieser ebenfalls mit Fructose durchgeführt. Besonderes Augenmerk wurde bei der Analytik auf sich bildende Reaktionsprodukte gelegt, die oftmals bereits in geringen Mengen für eine Farbbildung sowie für "off-taste" (unerwünschte Geschmackskomponenten) verantwortlich sind.

### Testbedingungen:

Modelllösung A: Palatinoselösung (10%ig) + 0,5% Carnitin
Modelllösung B: Fructoselösung (10%ig) + 0,5% Carnitin
Temperatur: 80°C
Erhitzungsdauer: 2 h

Die HPLC-Analyse bestätigte, dass die Isomaltuloselösung mit Carnitin als chemisch sehr stabil angesehen werden kann. Die durch den Test sich bildenden Nebenprodukte lagen bei 0,007 g/100ml. Die unter gleichen Bedingungen behandelte Fructoselösung mit Carnitin zeigte einen deutlich erhöhten Wert an Nebenprodukten, der bei 0,031 g/100ml lag.

Diese Daten zeigen, dass sich Isomaltulose für die Herstellung von Produkten, die Carnitin enthalten, besonders gut eignet, da deren Stabilität überraschenderweise deutlich höher als von Vergleichs-L-Carnitin-Lösungen ist.

### Beispiel 3: Rezeptur für ein Orangen-Getränk

Isomaltulose-Instant-Getränk Orange mit L-Carnitin

| Pos. | Zutaten | Anteil |
|---|---|---|
| 1 | Palatinose™ | 92,56 % |
| 2 | Zitronensäure (wasserfrei) | 4,96 % |
| 3 | Tri-Natrium-Citrat | 0,26 % |
| 4 | Tri-Calcium-Phosphat | 0,22 % |
| 5 | Vitamin C | 0,24 % |
| 6 | Trübungsmittel mit Farbstoff E 171 | 0,48 % |
| 7 | Farbstoff E 102 (85 %) | 0,01 % |
| 8 | Farbstoff E 110 (85 %) | 0,016 % |
| 9 | Gummi Arabicum (sprühgetrocknet) E 414 | 0,10 % |
| 10 | Xanthan E 415 | 0,10 % |
| 11 | Na-Carboxymethylcellulose E 466 | 0,10 % |
| 12 | Orangen-Aroma Typ 100 | 0,64 % |
| 13 | Orangen-Aroma Typ 120 | 0,24 % |
| 14 | Sucralose | 0,03 % |
| 15 | L-Carnitin | 0,04 % |
| Summe | | 100,0 % |

### Beispiel 4: Rezeptur für ein Sportlergetränk

Isomaltulose-Sport-Drink mit L-Carnitin

| Pos. | Zutaten | Anteil |
|---|---|---|
| 1 | Palatinose™ | 89, 84 % |
| 2 | Zitronensäure (wasserfrei) | 6,360 % |
| 3 | Vitamin C | 0,550 % |
| 4 | Tri-Natrium-Citrat | 1,194 % |
| 5 | Trübungsmittel mit Farbstoff E 171 | 0,262 % |
| 6 | Xanthan E 415 | 0,091 % |
| 7 | Na-Carboxymethylcellulose E 466 | 0,091 % |
| 8 | Sucralose | 0,300 % |
| 9 | Farbstoff E 102 (85 %) | 0,018 % |
| 10 | Aroma Grapefruit-Lemon | 1,090 % |
| 11 | L-Carnitin | 0,200 % |
| Summe | | 100,00 % |

### Beispiel 5: Rezeptur für ein ACE-Getränk

Isomaltulose-ACE-Drink mit L-Carnitin

| Pos. | Zutaten | Anteil |
|---|---|---|
| 1 | Palatinose™ | 94,50 % |
| 2 | Zitronensäure (wasserfrei) | 3,88 % |
| 3 | Tri-Natrium-Citrat | 0,27 % |
| 4 | Tri-Calcium-Phosphat | 0,25 % |
| 5 | Trübungsmittel mit Farbstoff E 171 | 0,30 % |
| 7 | Farbstoff E 110 (85 %) | 0,033 % |
| 8 | Farbstoff E 102 (85 %) | 0,0125% |
| 9 | Farbstoff Coffeebrown TF 8 | 0,0025% |
| 10 | Farbstoff E 129 (Allura Red) | 0,0015% |
| 11 | Vitamin E | 0,02 % |
| 12 | Provitamin A | 0,032 % |
| 13 | Xanthan E 415 | 0,170 % |
| 14 | Na-Carboxymethylcellulose E 466 | 0,170 % |
| 15 | Multifrucht-Aroma | 0,330 % |
| 16 | L-Carnitin | 0,020 % |
| Summe | | 100,00% |

## Patentansprüche

1. Gemisch, enthaltend L-Carnitin und mindestens ein niedrig-glykämisches Saccharoseisomer, wobei das Gemisch 0,05 bis 5 Gew.-% L-Carnitin und 5 bis 99,9 Gew.-% des niedrig glykämischen Saccharoseisomers (jeweils bezogen auf Gesamttrockensubstanz des Gemisches) enthält und wobei das niedrig-glykämische Saccharoseisomer Isomaltulose oder Leukrose ist.

2. Gemisch nach Anspruch 1, wobei das Gemisch 0,05 bis 5 Gewichts-% L-Carnitin und 10 bis 99,9 Gewichts-% niedrig-glykämisches Saccharoseisomer (jeweils bezogen auf Gesamttrockensubstanz des Gemisches) enthält.

3. Gemisch nach einem der Ansprüche 1 oder 2, wobei das Gemisch mindestens einen Zusatzstoff enthält.

4. Gemisch nach einem der vorhergehenden Ansprüche, wobei der mindestens eine Zusatzstoff ein Präbiotikum, ein Probiotikum, ein Ergänzungsstoff, eine fetthaltige Komponente, ein Milcherzeugnis oder ein süßendes Agens ist.

5. Gemisch nach einem der vorhergehenden Ansprüche, wobei das süßende Agens ein Zucker, ein Intensivsüßstoff oder ein Zuckeraustauschstoff ist.

6. Gemisch nach einem der vorhergehenden Ansprüche, wobei das Präbiotikum Inulin, Oligofructose und/oder Galactooligosaccharide ist (sind).

7. Gemisch nach einem der vorhergehenden Ansprüche, wobei der Intensivsüßstoff ausgewählt ist aus der Gruppe bestehend aus Sucralose, Natriumcyclamat, Acesulfam K, Neohesperidin-Dihydrochalkon, Glycyrrhizin, Steveosid, Monellin, Thaumatin, Aspartam, Dulcin, Saccharin, Naringin-Dihydrochalkon, Neotame und einer Mischung zweier oder mehrerer davon.

8. Gemisch nach einem der vorhergehenden Ansprüche, wobei der Zuckeraustauschstoff ausgewählt ist aus der Gruppe bestehend aus Isomalt, 1,1-GPM (1-O-α-D-Glucopyranosyl-D-mannit), 1,6-GPS (6-O-α-D-Glucopyranosyl-D-sorbit), 1,1-GPS (1-O-α-D-Glucopyranosyl-D-sorbit), Maltodextrine, Lactit, Maltit, Erythrit, Xylit, Mannit, Sorbit, Maltitsirup, hydrierte und nichthydrierte Stärkehydrolysate und einer Mischung zweier oder mehrerer davon.

9. Gemisch nach einem der vorhergehenden Ansprüche, wobei der mindestens eine Zusatzstoff ein Zucker, insbesondere Saccharose, Glucose, Fructose, Lactose, Maltose oder eine Mischung zweier oder mehrerer davon ist.

10. Gemisch nach einem der vorhergehenden Ansprüche, wobei der mindestens eine Zusatzstoff ein Milcherzeugnis, insbesondere ein Lactose-freies Milcherzeugnis ist.

11. Gemisch nach einem der vorhergehenden Ansprüche, wobei das Milcherzeugnis Magermilchpulver, Vollmilchpulver, Lactose-freies Magermilchpulver, Lactose-freies Vollmilchpulver, ein Molkenerzeugnis oder eine Mischung zweier oder mehrerer davon ist.

12. Gemisch nach einem der vorhergehenden Ansprüche, wobei der mindestens eine Zusatzstoff ein Ergänzungsstoff ist, ausgewählt ist aus der Gruppe bestehend aus Malzextrakt, Aromastoffen, Farbstoffen, Geschmacksstoffen, Fließmittel, Mineralstoffen wie Natrium und Calzium, insbesondere Salzen wie Natriumchlorid, Vitaminen, Folsäure, Emulgatoren, Ballaststoffen, Lecithin, Omega-3-Fettsäuren, Triglyzeride mittlerer Kettenlänge, Phythoöstrogene und Ascorbinsäuresalze.

13. Gemisch nach einem der Ansprüche 4 bis 12, wobei das Probiotikum Lactobacillen oder Bifidobakterien sind.

14. Gemisch nach einem der vorhergehenden Ansprüche, wobei das niedrig-glykämische Saccharoseisomer das einzige und alleinig in dem Gemisch vorkommende körpergebende süßende Agens ist.

15. Gemisch nach einem der vorhergehenden Ansprüche, wobei das niedrig-glykämische Saccharoseisomer der einzige und alleinig in dem Gemisch vorkommende Zucker ist.

16. Gemisch nach einem der vorhergehenden Ansprüche, wobei das niedrig-glykämische Saccharoseisomer das einzige und alleinig in dem Gemisch vorkommende süßende Agens ist.

17. Gemisch nach einem der vorhergehenden Ansprüche, wobei mehrere Zusatzstoffe vorhanden sind.

18. Gemisch nach einem der vorhergehenden Ansprüche, wobei das Gemisch 50 bis 75 Gew.-% (bezogen auf das Gesamttrockengewicht des Gemisches) niedrig-glykämisches Saccharoseisomer enthält.

19. Gemisch nach einem der vorhergehenden Ansprüche, wobei das Gemisch 1 bis 5 Gew.-% L-Carnitin (bezogen auf das Gesamttrockengewicht des Gemisches) enthält.

20. Produkt, wobei das Produkt ein Nahrungs-, Genuss- oder Arzneimittel ist und wobei das Nahrungs-, Genuss- oder Arzneimittel 20 bis 99 Gewichts-% eines Gemisches nach einem der Ansprüche 1 bis 19 enthält (bezogen auf Gesamttrockensubstanz des Produktes).

21. Produkt nach Anspruch 20, wobei das Produkt eine Tablette, ein Komprimat, ein Energieriegel, ein Kaugummi, eine Hartkaramelle, eine Weichkaramelle, eine Schokolade, ein Keks, eine Backware, ein Schokoriegel, ein Müsliriegel, ein Cerealienprodukt, eine Kaffee-, Tee- oder Kakaoinstantformulierung ist.

22. Produkt, hergestellt aus und enthaltend ein Gemisch gemäß einem der Ansprüche 1 bis 19 und ein Lösemittel, wobei das Produkt ein Getränk ist und das Getränk 2 bis 40 Gewichts-% eines Gemisches nach einem der Ansprüche 1 bis 19 enthält (bezogen auf das Gesamtgewicht des Getränkes).

23. Produkt nach Anspruch 22, wobei das Getränk ein Sportlergetränk, ein Cola-Getränk, ein Energiegetränk, eine enterale Formulierung oder ein Erfrischungsgetränk ist.

24. Produkt nach einem der Ansprüche 20 bis 23, enthaltend mindestens einen Zusatzstoff.

25. Verwendung von einem niedrig-glykämischen Saccharoseisomer in einem L-Carnitin-haltigen Produkt, insbesondere nach einem der Ansprüche 20 bis 24, zur Geschmacksverbesserung oder Geschmacksmaskierung des in dem Produkt vorhandenen L-Carnitins, wobei das niedrig-glykämische Saccharoseisomer Isomaltulose oder Leukrose ist.

26. Verwendung von einem niedrig-glykämischen Saccharoseisomer in einem L-Carnitin-haltigen Produkt, insbesondere nach einem der Ansprüche 20 bis 24, zur Erhöhung der Stabilität von L-Carnitin in dem Produkt, wobei das niedrig-glykämische Saccharoseisomer Isomaltulose oder Leukrose ist.

## Claims

1. Mixture, containing L-carnitine and at least one low-glycemic sucrose isomer, wherein the mixture contains 0.05 to 5% by weight L-carnitine and 5 to 99.9% by weight low-glycemic sucrose isomer (each based on the total dry solids of the mixture), and wherein the low-glycemic sucrose isomer is isomaltulose or leucrose.

2. Mixture according to claim 1, wherein the mixture contains 0.05 to 5% by weight L-carnitine and 10 to 99.9% by weight low-glycemic sucrose isomer (each based on the total dry solids of the mixture).

3. Mixture according to any one of claims 1 or 2, wherein the mixture contains at least one additive.

4. Mixture according to any one of the preceding claims, wherein the at least one additive is a prebiotic, a probiotic, a supplement, a fat-based ingredient, a dairy product or a sweetening agent.

5. Mixture according to any one of the preceding claims, wherein the sweetening agent is a sugar, an intense sweetener or a sugar substitute.

6. Mixture according to any one of the preceding claims, wherein the prebiotic is inulin, oligofructose and/or galactooligosaccharides.

7. Mixture according to any one of the preceding claims, wherein the intense sweetener is selected from the group comprising sucralose, sodium cyclamate, acesulfame K, neohesperidine dihydrochalcone, glycyrrhizine, stevioside, monellin, thaumatin, aspartame, dulcin, saccharine, naringindihydrochalcone, neotame, and a mixture of two or more of these.

8. Mixture according to any one of the preceding claims, wherein the sugar substitute is selected from the group comprising isomalt, 1,1-GPM (1-O-α-D-glucopyranosyl-D-mannitol), 1,6-GPS (6-O-α-D-glucopyranosyl-D-sorbitol), 1,1-GPS (1-O-α-D-glucopyranosyl-D-sorbitol), maltodextrins, lactitol, maltitol, erythritol, xylitol, mannitol, sorbitol, maltitol syrup, hydrogenated and non-hydrogenated starch hydrolysates, and a mixture of two or more of these.

9. Mixture according to any one of the preceding claims, wherein the at least one additive is a sugar, in particular sucrose, glucose, fructose, lactose, maltose or a mixture of two or more of these.

10. Mixture according to any one of the preceding claims, wherein the at least one additive is a dairy product, in particular a lactose-free dairy product.

11. Mixture according to any one of the preceding claims, wherein the dairy product is skim milk powder, whole milk powder, lactose-free skim milk powder, lactose-free whole milk powder, a whey product or a mixture of two or more of these.

12. Mixture according to any one of the preceding claims, wherein the at least one additive is a supplement selected from the group comprising malt extract, flavoring substances, dyes, flavorings, flow aids, minerals such as sodium and calcium, in particular salts such as sodium chloride, vitamins, folic acid, emulsifiers, dietary fiber, lecithin, omega-3 fatty acids, medium-chain-length triglycerides, phytoestrogens, and ascorbic acid salts.

13. Mixture according to any one of claims 4 to 12, wherein the probiotic is Lactobacilli or Bifidobacteria.

14. Mixture according to any one of the preceding claims, wherein the low-glycemic sucrose isomer is the only bodying sweetening agent present in the mixture.

15. Mixture according to any one of the preceding claims, wherein the low-glycemic sucrose isomer is the only sugar present in the mixture.

16. Mixture according to any one of the preceding claims, wherein the low-glycemic sucrose isomer is the only sweetening agent present in the mixture.

17. Mixture according to any one of the preceding claims, wherein several additives are present.

18. Mixture according to any one of the preceding claims, wherein the mixture contains 50 to 75% by weight (based on the total dry weight of the mixture) low-glycemic sucrose isomer.

19. Mixture according to any one of the preceding claims, wherein the mixture contains 1 to 5% by weight L-carnitine (based on the total dry weight of the mixture).

20. Product, wherein the product is a food, luxury food or drug, and wherein the food, luxury food or drug contains 20 to 99% by weight of a mixture according to any one of claims 1 to 19 (based on the total dry weight of the mixture).

21. Product according to claim 20, wherein the product is a tablet, a compressed product, an energy bar, a chewing gum, a hard caramel, a soft caramel, a chocolate, a cookie, a baked good, a chocolate bar, a granola bar, a cereal product, a coffee, tea or cocoa instant formulation.

22. Product, produced from and containing a mixture according to any one of claims 1 to 19, and a solvent, wherein the product is a beverage, and wherein the beverage contains 2 to 40% by weight of a mixture according to any one of claims 1 to 19 (based on the total weight of the beverage).

23. Product according to claim 22, wherein the beverage is a sports beverage, a beverage containing cola, an energy drink, an enteral formulation or a refreshment beverage.

24. Product according to any one of claims 20 to 23 containing at least one additive.

25. Use of a low-glycemic sucrose isomer in a product containing L-carnitine, in particular according to any one of claims 20 to 24, to improve the flavor or to mask the flavor of the L-carnitine present in the product, wherein the low-glycemic sucrose isomer is isomaltulose or leucrose.

26. Use of a low-glycemic sucrose isomer in a product containing L-carnitine, in particular according to any one of claims 20 to 24, to increase the stability of L-carnitine in the product, wherein the low-glycemic sucrose isomer , is isomaltulose or leucrose.

## Revendications

1. Mélange contenant de la L-carnitine et au moins un isomère de saccharose à faible indice glycémique, lequel mélange contient 0,05 à 5 % en poids de L-carnitine et de 5 à 99,9 % en poids de l'isomère de saccharose à faible indice glycémique (rapporté à chaque fois à la matière sèche totale du mélange) et l'isomère de saccharose à faible indice glycémique étant un isomaltulose ou un leucrose.

2. Mélange selon la revendication 1, lequel mélange contient 0,05 à 5 % en poids de L-carnitine et de 10 à 99,9 % en poids d'un isomère de saccharose à faible indice glycémique (rapporté à chaque fois à la matière sèche totale du mélange).

3. Mélange selon l'une quelconque des revendications 1 ou 2, lequel mélange contient au moins un additif.

4. Mélange selon l'une quelconque des revendications précédentes, dans lequel le au moins un additif est un prébiotique, un probiotique, un complément alimentaire, un composant présentant une teneur en matière grasse, un produit laitier ou un agent sucré.

5. Mélange selon l'une quelconque des revendications précédentes, dans lequel l'agent sucré est un sucre, un édulcorant à fort pouvoir sucrant ou un succédané du sucre.

6. Mélange selon l'une quelconque des revendications précédentes, dans lequel le prébiotique est l'inuline, l'oligofructose et/ou le galactooligosaccharide.

7. Mélange selon l'une quelconque des revendications précédentes, dans lequel l'édulcorant à fort pouvoir sucrant est sélectionné dans le groupe consistant en du sucralose, du cyclamate de sodium, l'acésulfam K, de la néohespéridine dihydrochalcone, de la glycyrrhizine, du stévioside, de la monelline, de la thaumatine, de l'aspartame, de la dulcine, de la saccharine, de la naringine dihydrochalcone, du néotame et un mélange de deux ou plus de ces substances.

8. Mélange selon l'une quelconque des revendications précédentes, dans lequel le succédané du sucre est sélectionné dans le groupe consistant en de l'isomalt, du 1,1-GPM (1-O-α-D-glucopyranosyl-D-mannitol), du 1,6-GPS (6-O-α-D-glucopyranosyl-D-sorbitol), du 1,1-GPS (1-O-α-D-glucopyranosyl-D-sorbitol), de la maltodextrine, du lactitol, du maltitol, de l'érythritol, du xylitol, du mannitol, du sorbitol, du sirop de maltitol, des hydrolysats d'amidon hydrogénés et non hydrogénés et un mélange de deux ou plus de ces substances.

9. Mélange selon l'une quelconque des revendications précédentes, dans lequel le au moins un additif est un sucre, notamment un saccharose, un glucose, un fructose, un lactose, un maltose ou un mélange de deux ou plus de ces substances.

10. Mélange selon l'une quelconque des revendications précédentes, dans lequel le au moins un additif est un produit laitier, notamment un produit laitier ne contenant pas de lactose.

11. Mélange selon l'une quelconque des revendications précédentes, dans lequel le produit laitier est une poudre de lait maigre, une poudre de lait entier, une poudre de lait maigre sans lactose, une poudre de lait entier sans lactose, un lactosérum ou un mélange de deux ou plus de ces substances.

12. Mélange selon l'une quelconque des revendications précédentes, dans lequel le au moins un additif est un complément alimentaire, sélectionné dans le groupe consistant en de l'extrait de malt, des substances aromatiques, des colorants, des exhausteurs de goût, des fluidifiants, des substances minérales telles que du sodium et du calcium, notamment des sels tels que du chlorure de sodium, des vitamines, de l'acide folique, des émulsifiants, des fibres alimentaires, de la lécithine, des acides gras oméga 3, des triglycérides de longueur de chaîne moyenne, des phytooestrogènes et des sels d'acide ascorbique.

13. Mélange selon l'une quelconque des revendications 4 à 12, dans lequel le probiotique consiste en des lactobacilles ou des bifidobactéries.

14. Mélange selon l'une quelconque des revendications précédentes, dans lequel l'isomère de saccharose à faible indice glycémique est le seul et unique agent sucrant alimentant le corps présent dans le mélange.

15. Mélange selon l'une quelconque des revendications précédentes, dans lequel l'isomère de saccharose à faible indice glycémique est le seul et unique sucre présent dans le mélange.

16. Mélange selon l'une quelconque des revendications précédentes, dans lequel l'isomère de saccharose à faible indice glycémique est le seul et unique agent sucrant présent dans le mélange.

17. Mélange selon l'une quelconque des revendications précédentes, dans lequel plusieurs additifs sont présents.

18. Mélange selon l'une quelconque des revendications précédentes, lequel mélange contient de 50 à 75 % en poids (rapporté à chaque fois au poids sec total du mélange) d'un isomère de saccharose à faible indice glycémique.

19. Mélange selon l'une quelconque des revendications précédentes, lequel mélange contient de 1 à 5 % en poids de L-carnitine (rapporté à chaque fois au poids sec total du mélange).

20. Produit, lequel produit est une denrée alimentaire, une denrée de luxe ou un médicament et laquelle denrée alimentaire, laquelle denrée de luxe ou lequel médicament contient 20 à 99 % en poids d'un mélange selon l'une quelconque des revendications 1 à 19 (rapporté à chaque fois à la matière sèche totale du produit).

21. Produit selon la revendication 20, lequel produit est un comprimé, un cachet, une barre énergétique, une gomme à mâcher, un caramel dur, un caramel mou, un chocolat, un gâteau, une pâtisserie, une barre chocolatée, une barre de céréales, un produit céréalier, une composition instantanée pour du café, du thé ou du cacao.

22. Produit fabriqué à partir de et contenant un mélange selon l'une des revendications 1 à 19 et un agent de dissolution, lequel produit est une boisson et la boisson contient de 2 à 40 % en poids d'un mélange selon l'une quelconque des revendications 1 à 19 (rapporté à chaque fois au poids total de la boisson).

23. Produit selon la revendication 22, laquelle boisson est une boisson pour les sportifs, une boisson à base de cola, une boisson énergétique, une formulation entérale ou une boisson rafraîchissante.

24. Produit selon l'une quelconque des revendications 20 à 23, contenant au moins un additif.

25. Utilisation d'un isomère de saccharose à faible indice glycémique dans un produit contenant de la L-carnitine, notamment selon l'une quelconque des revendications 20 à 24, destinée à améliorer la saveur ou à masquer le goût de la L-carnitine présente dans le produit, dans lequel l'isomère de saccharose à faible indice glycémique est un isomaltulose ou leucrose.

26. Utilisation d'un isomère de saccharose à faible indice glycémique dans un produit contenant de la L-carnitine, notamment selon l'une quelconque des revendications 20 à 24, destinée à accroître la stabilité de la L-carnitine dans le produit, dans lequel l'isomère de saccharose à faible indice glycémique est un isomaltulose ou leucrose.
